Europäisches Patentamt

European Patent Office

Office européen des brevets

(11) Publication number: **0 267 776**
**A2**

## (12) EUROPEAN PATENT APPLICATION

(21) Application number: **87309920.4**

(22) Date of filing: **10.11.87**

(51) Int. Cl.⁴: **A 61 M 5/32**

(30) Priority: **10.11.86 GB 8626763**
**03.04.87 GB 8708059**

(43) Date of publication of application:
**18.05.88 Bulletin 88/20**

(84) Designated Contracting States:
**AT BE CH DE ES FR GB GR IT LI LU NL SE**

(71) Applicant: **A.C. DANIELS & CO.,LTD.,**
**130 Western Road**
**Tring Hertfordshire HP23 4BU (GB)**

(72) Inventor: **Hayward, Ronald F.**
**Molloway House Dunsmore**
**Wendover Bucks HP22 6QJ (GB)**

(74) Representative: **Milhench, Howard Leslie et al**
**R.G.C. Jenkins & Co. 26 Caxton Street**
**London SW1H 0RJ (GB)**

(54) **Vessel for disposal of surgical waste.**

㊗ A vessel for containing surgical waste comprising an upper container part (10), a lower container part (12), a locking pin (14), a strap (16), and a sliding door (18). The upper container part and the lower container part are supplied to the user separately and include a joining arrangement which unreleasably locks and seals the two parts together. The upper container part includes an access opening (20) which is covered by the door (18). The door can be unreleasably closed by inserting the locking pin (14) into a locking pin hole (24) when the door (18) is closed.

FIG.1.

EP 0 267 776 A2

**Description**

VESSEL FOR DISPOSAL OF SURGICAL WASTE

This invention relates to a vessel for safely containing contaminated surgical waste from operating theatres, intensive care and other hospital units, and the like.

Scalpels, hypodermic syringes and other medical devices, collectively known as sharps, become contaminated during use and must be disposed of safely and securely. During an operation, or during the normal routine for example of a hospital unit, sharps and other potentially dangerous and infective waste is generated and must be safely stored for disposal, not only to protect surgical and allied staff but also to protect the personnel whose task it is to clear the waste and attend to its disposal. Such other waste may include liquid wastes in blood laden swabs and partially exhausted syringes for example and therefore, in order to achieve secure containment of all categories of waste, the vessel used should desirably be designed not only to resist penetration by sharps but also to protect against fluid leakages.

Surgical waste disposal vessels have previously been relatively bulky, requiring large storage areas within the confines of a surgically sterile environment, and moreover have commonly been provided with normally-closed spring-loaded or gravity biassed access flaps which give rise to a risk of injury and contamination on account of the fact that waste cannot simply be dropped into the vessel. In addition, such vessels are subject to being knocked over in the course of intensive activity during an operation, and hitherto have not been sufficiently protected against spillage of fluid from the vessel. Generally speaking, insufficient attention has been given in the past to the question of secure disposal of surgical wastes.

The object of the present invention is to overcome or at least substantially reduce these problems, particularly having regard to the virulence of the so-called Aids virus and the concomitant extreme risk of infection, and to provide a safer, more secure and more economical means for containing sharps and other surgical waste before incineration.

As will be described hereinafter the above object is attained by a disposable vessel for containing surgical waste comprising a first and second container parts formed of synthetic plastics material, the first container part including access means for receiving surgical waste, and both of the container parts having fastening means for unreleasably fastening the two container parts together in a fashion which is substantially leakage proof as regards liquid wastes.

More particularly, in accordance with one of its aspects the present invention provides a vessel for use in the disposal of surgical waste, the said vessel comprising first and second parts with one at least of said parts constituting a container and the other of said parts constituting a closure for said container, said first and second parts being adapted to be secured to each other so as in use to be substantially incapable of accidental detachment one from the other, and the closure part having an access opening providing direct access to the container part when the two parts are secured to each other and having furthermore a releasable closure for said access opening.

The invention in a first preferred embodiment provides a vessel which is substantially rigid when assembled so that wastes cannot readily be tipped out of the vessel simply by distorting one of the container parts relative to the other, has a single opening designed to allow the user simply to drop waste materials into the container without need to place his hand in contact with the container, is supplied in two generally similar halves which can be stacked during storage and may be quickly and easily assembled in such a way that subsequent dismantling or re-use is virtually impossible, and wherein the vessel parts include sealing means co-operating to inhibit leakage of fluid waste from the vessel when the two parts of the vessel are fastened together for use.

In one exemplary embodiment which is described in detail hereinafter, the fastening means is comprised by a matching plurality of barbed lugs and holes provided on the peripheries of the open ends of the container parts, the barbed lugs of one part being adapted to inter-engage with the corresponding holes of the other part on assembly of the two parts with each other, and the sealing means is comprised by a face-sealing rim at the perimeter of the open end of each container and flange means sealing the junction of the peripheral face seals, such flange means advantageously comprising a plurality of overlapping flanges spaced around the outer edge of the rim and arranged so that the flanges on one container part cooperate with the outer edge of the rim on the other container part to form a seal. The rim of each container part is advantageously supported by a plurality of ribs to strengthen the assembled vessel.

In accordance with other embodiments described hereinafter, rather than the attachment of the two container parts being by virtue of barbed lugs engageable in receptor-holes and the sealing being by virtue of face seals and co-operating flanges, instead both the attachment and the sealing is effected by virtue of the inherent resilience of the synthetic plastics material of which the container parts are preferably formed. Thus in one arrangement hereinafter described, one of the container parts is formed with an annular peripheral recess into which a generally complementary-shaped annular peripheral formation provided on the other container part is adapted to be snap-fitted when the two parts are secured together and with the resilience of the formation effecting a seal within the recess. In another arrangement, the peripheral end of one container part is generally bell-shaped and the other container part has a turned-over peripheral rim adapted to be snap-fitted into the bell-shaped mouth of the first-mentioned part.

In yet another embodiment, a lid forming one container part is adapted to be snap-fitted to a box forming the other container part and the sealing of the two parts is achieved by means of a combination of face seals and labyrinthine paths.

Preferably, the access means includes means for unreleasably locking the vessel in closed condition in preparation for disposal of the vessel and its waste contents. Thus, in exemplary embodiments which are described in detail hereinafter, the access means is a door which can be slid across an access opening in one of the container parts, and the means for unreleasably locking the door is a locking pin which is insertable into an opening in the body of the vessel near the door, the locking pin having one or more formations inhibiting its removal once inserted so that it cannot be accidentally removed. The door may conveniently be pivoted for movement across the access opening, and the locking pin may conveniently be tied to the vessel by means of a strap secured at the pivot axis.

Preferably the vessel further includes means for temporarily locking the door over the access opening.

The door is desirably arranged to seal the periphery of the access opening when the door is across the access opening, thereby inhibiting leakage of fluid waste if the vessel is tipped over.

Further features of the present invention are set forth with particularity in the appended claims and, together with advantages of the invention, will be clearly understood from consideration of the following detailed description of exemplary emnodiments which are illustrated in the accompanying drawings in which:

FIGURE 1 is an exploded perspective view of a vessel embodying the present invention;

FIGURE 2 is a perspective view showing detail of connecting and locking means prior to assembly;

FIGURE 3 is a part sectional view showing how barbed lugs on one container part pass through slots in the other container part to lock the two parts together;

FIGURE 4 is a perspective detail of an access door and locking pin on a flat end surface of the vessel;

FIGURE 5 is a plan view of the assembled parts in FIGURE 4 looking into the open end of an upper container part;

FIGURE 6 is a perspective view of the assembled vessel;

FIGURE 7 is a side view showing how the two parts can be stacked with others of the same during storage;

FIGURE 8 is a view similar to that of FIGURE 3 but showing an alternative means of securing and sealing the two container parts together;

FIGURE 9 is a plan view of a lid assembly of a second embodiment of the present invention;

FIGURE 10 is a part sectional view showing how a rim on the lid engages with a rim on a vessel body of the second embodiment of the present invention; and

FIGURE 11 is a fragmented, part-sectional perspective view of a third embodiment of the invention.

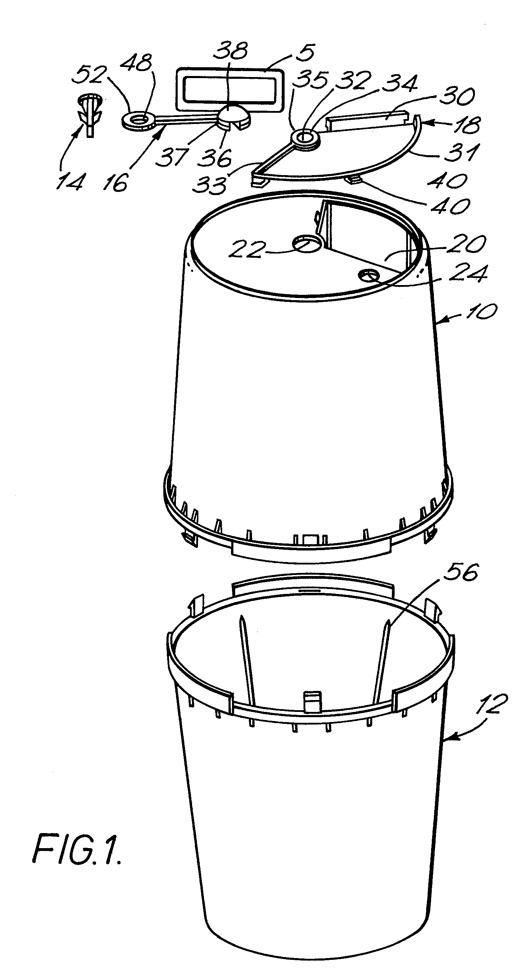

Referring to Figure 1 the waste disposal vessel shown comprises an upper container part 10, a lower container part 12, a locking pin 14, a strap 16 and a door 18, all formed of synthetic resin material. A carrying handle 5 can conveniently be formed integrally with the strap 16. The flat end panel 26 of the upper container part is formed with an access opening 20, a pivot hole 22 and a locking pin hole 24.

Ribs 56 on the inner surface of both the upper container part 10 and the lower container part 12 provide reinforcement of the relatively thin side walls of the vessel.

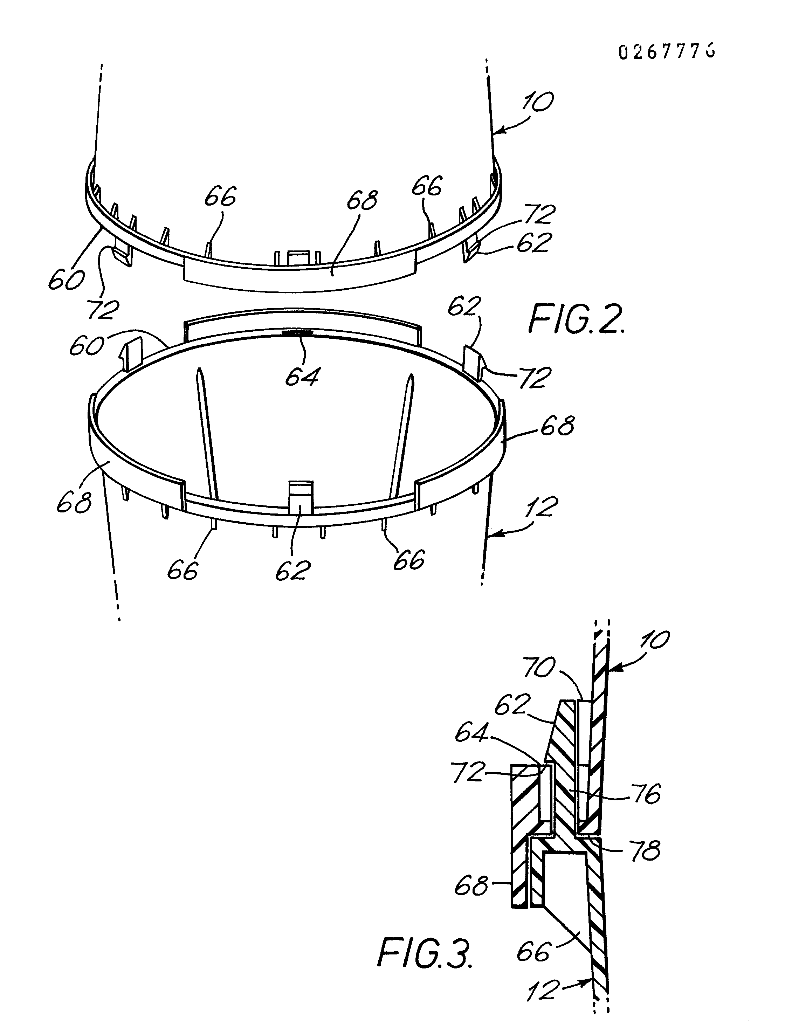

As shown in Figure 2, the open ends of the upper container part 10 and the lower container part 12 both include a rim 60 with a plurality of barbed lugs 62, and an equal plurality of receiving slots 64. The rim 60 is supported and strengthened by a plurality of supporting ribs 66 which extend between the rim 60 and the body of the container part. Overlap flanges 68 are also provided on the outer edge of the rim 60.

The upper and lower container parts 10, 12 are supplied separately and are assembled by the user. To assemble the vessel the barbed lugs 62 on one container part are aligned with corresponding receiving slots 64 on the other container part. Alignment of the barbed lugs 62 and receiving slots 64 is aided by the overlap flanges 68 on both container parts which are arranged so that an overlap flange on one container part will fit snugly into the gap on a portion of the rim between two overlap flanges on the other container part. When the two container parts have been aligned they are locked by pressing the two container parts together so that the barbed lugs 62 on one container part pass through corresponding receiving slots on the other container part, and vice versa.

Figure 3 shows how the barbed lugs 62 pass through the receiving slots 64 and cooperate with the underside 72 of the rim to lock the two container parts together. With the two container parts thus engaged, a cam 70 on the main body of the container part pushes the barbed lugs 62 away from the main body so that the latching surface 72 on the barbed lug 62 overlaps a ridge 74 on the under surface of the rim on the outer container part, thus preventing separation of the two container parts.

The length of the main shaft 76 of the barbed lug is substantially the same as the distance between the latching surface 72 and the main surface 78 of the rim, thus ensuring that the main surface of the rim on one container part contacts the main surface 80 of the rim on the other container part over a significant portion of the area of the rims so as to constitute a face seal between the two container parts. In addition the overlap flanges 68 come in contact with a significant portion of the outer edge surface 82 of the rim, thus constituting a further seal for further reducing the possibility of fluid waste leaking from the vessel.

Referring now to Figures 1, 4 and 5, the door 18 locates on the inner surface of the flat end panel 26 of vessel part 10 with the portion 30 projecting through the access opening 20 and extending radially, and the hole 32 in the pivot ring 34 registering coaxially with the pivot hole 22 in end panel 26. The pivot lugs 36 on the strap 16 snap fit through the pivot hole 22 and through the hole 32 in the pivot ring 34 on the door 18 to pivotally mount the door 18, and are retained in position by their projections 37. The under-surface of the dome 38 on the strap 16 and the pivot ring 34 on the door 18 co-operate with a portion of the flat panel peripheral to the pivot hole 22 to substantially seal the pivot hole 22, thereby reducing the possibility of fluid waste leaking through the pivot hole 22. Raised rims 31, 33, and 35 are provided around the periphery of the door on the same side as the projecting portion 30 and are held in contact with the periphery of the access opening 20 by means of lugs 40 on the door 18 which engage under a rib 42 provided on the inner surface of the upper container part 10 so as to urge the door 18 towards the panel 26; by virtue of this arrangement the door 18 is held in sealing contact with the inner surface of the end panel 26 when the door is moved across the access opening 20 to close the vessel.

The door 18 is firmly held in place by the pivot lugs 36 on the strap 16 and by means of the cooperation of the lugs 40 with the rib 42 on the inner surface of the container, and yet is readily pivotable between open and closed positions with the lugs 40 riding on the rib 42. As will be appreciated, the door action is not wasteful of space within the vessel and the door can readily be closed even when the vessel is full.

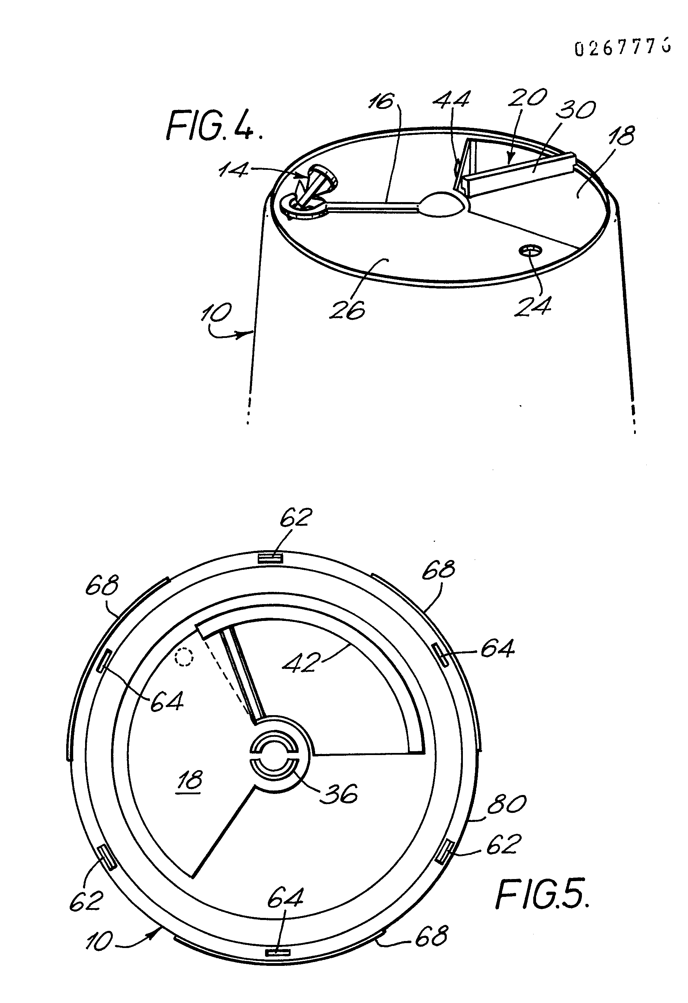

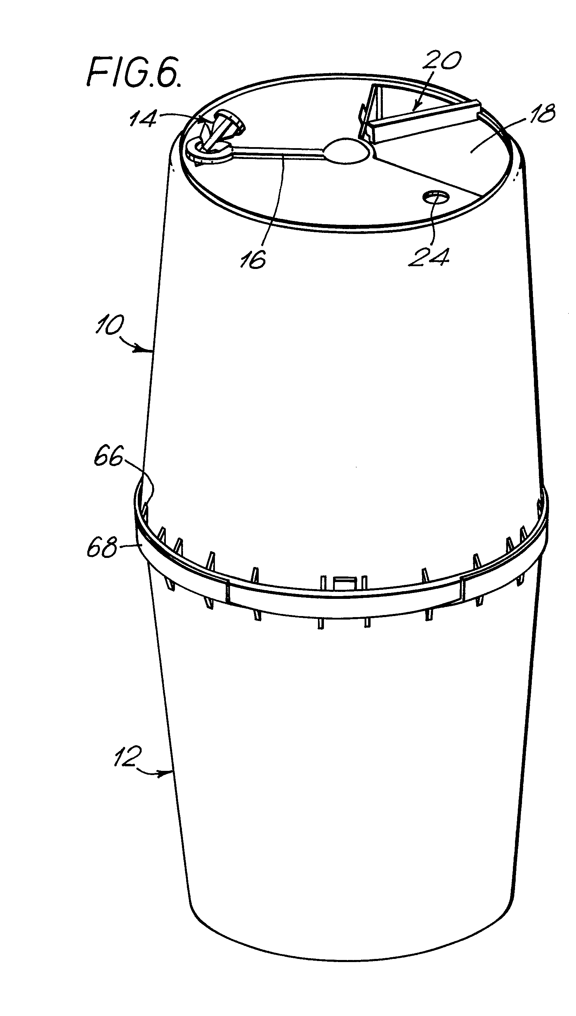

An upstanding tab 44 shown in Figure 4 is provided on the outer surface of the end panel 26 and is arranged to cooperate with a recess 46 shown in Figure 5 and formed in the under-surface of the door 18 below the projecting portion 30 to define a releasable latch for temporarily holding the door closed across the access opening 20. A gentle force by the user is all that is required to release the recess 46 from the tab 44 and allow the user to open the door. The access opening 20 is in the top of the vessel and is of such dimensions that when the door 18 is open the access opening is large enough for contaminated sharps and other waste material to be simply dropped into the vessel without the vessel having to be touched at all and yet is small enough that users cannot accidentally put a hand inside the vessel thus reducing the danger of injury and infection therefrom.

The locking pin 14 is received in a hole 48 at the free end of the strap 16 and accidental removal or loss of the pin from the strap is prevented by barbs 50 projecting from the pin 14.

When the vessel and its waste contents are to be disposed of, the door 18 is closed and locking pin 14 is inserted into the locking pin hole 24 to prevent the door from being reopened, accidental subsequent removal of the locking pin being inhibited by the barbs 50 co-operating with the periphery of the locking pin hole 24. The barbs 50 on the locking pin 16 serve furthermore to urge the ring portion 52 at the free end of the strap 16 against a corresponding portion of the upper end panel 26 so as substantially to seal the locking pin hole 24.

The upper and lower container parts can conveniently be made by an injection moulding process using a suitable synthetic plastic material and with the thickness of the walls of the vessel sufficient to ensure that disposed sharps cannot pierce the material. Since the upper and lower container parts are identical except for the openings in the end of the upper part, the two parts can be moulded using the same tooling with removable tool parts defining the requisite openings or with the openings being stamped into the respective parts after moulding.

An assembled vessel is shown in Figure 6. Separately, the two container parts have a degree of flexibility limited by the rim 60 and by the ribs 56 on their inner surfaces. Once the two container parts have been locked together however the two rims and associated flange portions cooperate to give the vessel much greater rigidity. As a result, the join between the two container parts is resistive to gaping apart under applied force thereby ensuring that the vessel remains sealed at the adjoining rims.

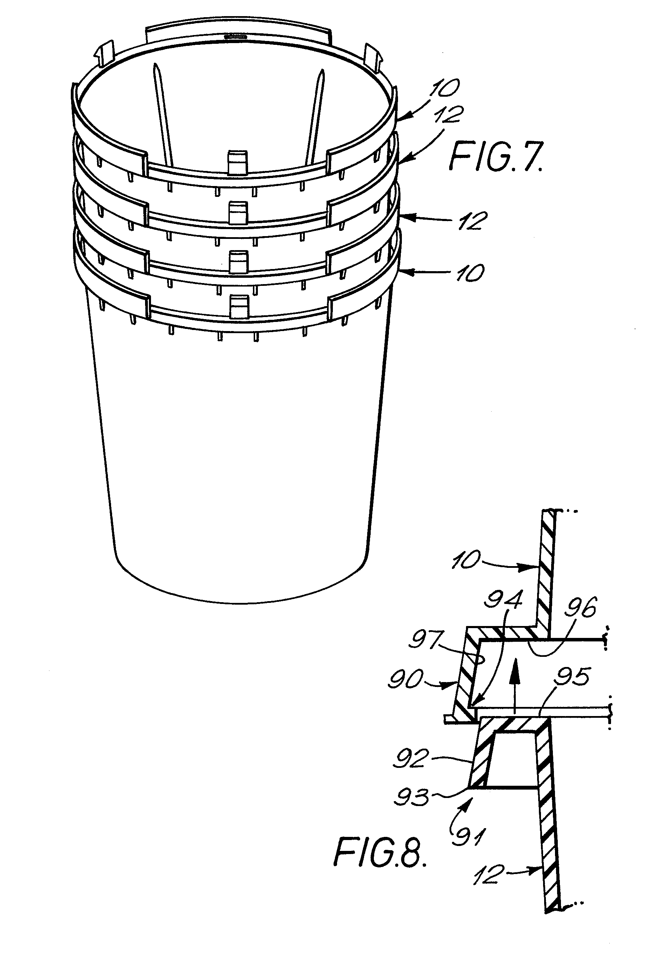

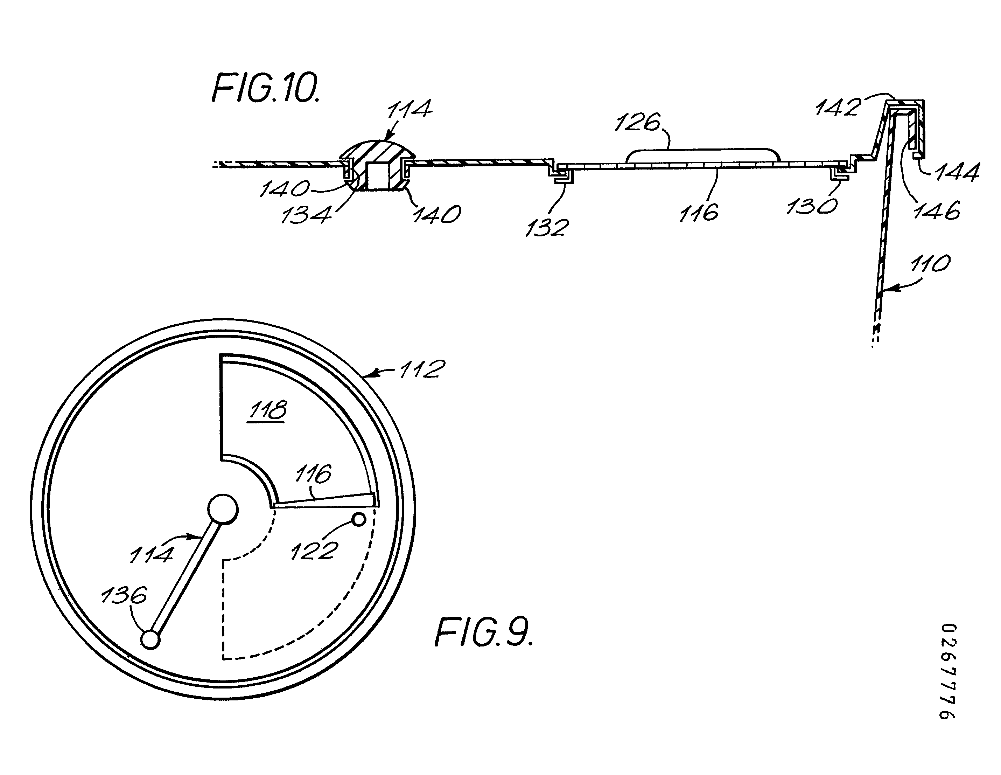

Referring to Figure 7, the upper and lower container parts have similar bucket-like form, allowing the two parts to be stacked together. In this way, a large number of vessels can be stored in a very small space and yet remain readily accessible for use.

It is apparent from the above description that by virtue of the invention a vessel for containing contaminated surgical sharps and other waste can be supplied to the user in two separate parts for efficient, convenient storage in minimum space, and can be easily assembled by aligning the two halves and pressing firmly until all the barbed lugs have engaged with the latching surfaces on the two container parts whereupon the two container parts cannot be separated without substantial difficulty. An access door at the top of the vessel can be opened and closed temporarily in use, and may be unreleasably locked when it is desired to dispose of the vessel and its contents. The two parts of the vessel seal together in substantially fluid tight manner, and the door also seals when closed.

Referring now to FIGURE 8, shown therein is a view similar to FIGURE 3 of a modified fastening and sealing arrangement for the two container parts 10 and 12. As shown, the container part 10, which otherwise may be as described hereinbefore, has an enlarged or bell-shaped mouth 90 into which a turned-over peripheral edge portion 91 of the container part 12 is adapted to be snap-fitted by forced movement of the container part 12 in the direction of the arrow. The outer surface 92 of the edge portion 91 of container part 12 is slightly curved and defines a sealing knife-edge 93 which is adapted to snap-fit and seal with a recess 94 defined in the bell-shaped mouth of the other container part. Furthermore the planar end face 95 is adapted to face seal with the opposed face 96 of container part 10 and likewise the surface 92 of container part 12 is adapted to face seal with the surface 97 of container part 10. The thus described annular formations at the peripheral edges of the two container parts co-operate not only to secure and seal the two parts together, but also constitute a strengthening ring about the middle of the assembled container. When the two parts of the container are assembled together, they cannot accidentally be separated and can be separated only with substantial difficulty.

Referring now to Figures 9 and 10, a second embodiment of the invention is a schematically illustrated surgical waste disposal vessel which comprises a main body 110 which can for example be generally cylindrical or bucket-shaped, a lid 112, a pivot point and locking pin element 114 and a door 116 is shown. The lid 112 is formed with an access opening 118, a pivot hole 120 and a locking pin hole 122. The door 116 includes a raised peripheral portion 124, a finger pull 126, locking aperture 128, an inner retaining lug 130 and an outer retaining lug 132, and a pivot hole 134.

The door 116 is secured to the lid 112 by an arrangement similar to that shown in Figures 1 and 5 of the first embodiment except that in the present case the door locates on the upper side of the lid. Lugs 140 on a pivot point part at one end of the locking pin element pass in snap fit manner through the hole 134 on the door 116 and through the pivot hole 20 so that projections on the lugs 140 act against the inner surface of the lid peripheral to the pivot hole 120 to pivotally secure the door 116 to the lid 112. The raised peripheral portion 124 is provided to increase the rigidity of the door, and the finger pull 126 is provided for ease of operation when opening or closing the door. The two retaining lugs 130, 132 cooperate with the rim of the access hole 118 to stabilize the door as it is moved across the access hole 118.

A locking pin 136 is integrally formed with the locking pin element 114 at the other end thereof. The locking pin can be substantially the same as the locking pin described in connection with the first embodiment and can be utilized for releasably latching the door across the access hole 118 in which case the pin is not pushed fully home, or alternatively for locking the door closed in which case the locking pin is pressed fully home so that barbs provided on the locking pin engage with the under surface of the lid around the locking pin hole 122 thereby making subsequent removal of the pin, and hence access to the contents of the vessel, difficult.

A rim 142 is provided around the periphery of the lid 112 and includes a rib 144 which projects inwardly. Upon assembly, the rim 142 engages sealingly with the lip 146 of the main body 110. The lip 146 cooperates with the rib 144 so that subsequent separation of the lid and the main body is made difficult and accidental separation is substantially impossible.

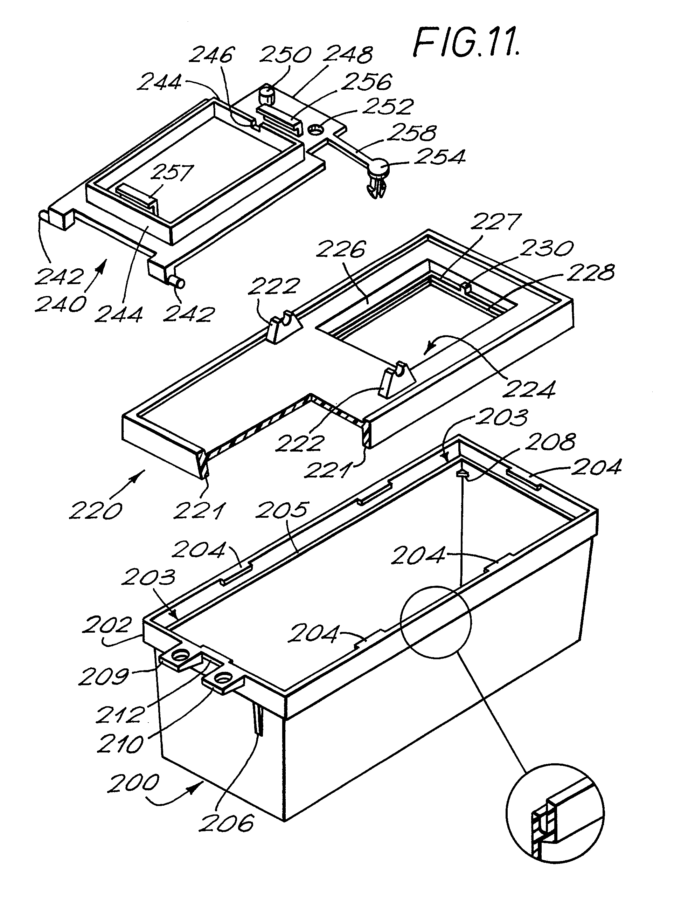

A third embodiment will now be described with reference to FIGURE 11 of the accompanying drawings which shows an isometric view prior to assembly of a vessel for containing surgical waste and which is designed to fit into a doctor's or nurse's visiting case.

The vessel comprises a body 200, a lid 220, and an access door 240. In the drawing the access door 240 is shown as viewed from underneath. The body 200 comprises a rim 202 extending outwards and upwards away from the body slightly below the upper edge 205 of the body as is shown clearly by the scrap sectional view. A plurality of retaining lugs 204 co-operate with the periphery of the upper surface of the lid 220 to lock the lid into position over the opening in the body on assembly. The rim being thus arranged, a channel 203 is formed between the upper edge 205 of the body and the inner edge of the rim. Upon assembly, a peripheral flange 221 on the lower surface of the lid projects down into the channel 203 to form a snug, substantially waterproof labyrinthine seal between the body 200 and the lid 220. A plurality of supporting ribs 206 are provided at suitable intervals to support the rim and to provide rigidity to the body, and additional internal supporting ribs 208 are provided on the internal corners of the body to provide additional rigidity to the body. On one end of the body 200 at or near the top of the rim 202 two holed tabs 209,210, are provided, the function of which will be explained hereinafter.

The lid 220 comprises two door supports 222 which engage with corresponding pivots 242, positioned at one end of the access door 240 to provide a hingeing action whereby access may be obtained to the vessel in use. The lid 220 includes an access opening 224 defined by an inner edge 226 which supports an inner rim 228 extending inwards and upwards away from the inner edge 226. The inner edge 226 and the inner rim 228 are so arranged to provide a channel 227 which co-operates with a projection 244 on the access door 240 to seal the access opening 224 when the lid is closed. An engagement stud 230 positioned in the channel 227 is arranged so that a cut-away section 246 in the projection 244 on the lid 240 clips over the engagement stud 230 to hold the access door over the access opening when the access door is in the closed position. The access door 240 further comprises a gripping portion 248 which includes a locating stud 250, a locking hole 252, clip members 256 and 257, and a locking pin 254 secured to the handle portion by a strap 258.

In use the access door is hinged about the pivots 242 so that the user may quickly and easily open the access door to dispose of spent syringes etc. To ensure that the access door 240 remains closed over the access opening at other times the clip member 256 is arranged so as to engage with a projection 212 on the body between the holed tabs 209,210. Moreover, the locating stud 250 is positioned such that it will be automatically inserted into the hole in the holed tab 209 on the body when the access door is closed and is dimensioned to provide a snug fit. Once the vessel is full, or at any other suitable time prior to disposal, the locking pin 254 may be inserted through the locking hole 252 and through the hole in the hole tab 210 to lock the access door down over the access hole. As in the previously described embodiments, the locking pin, once inserted into its accommodating holes, cannot release accidentally.

The body 200 may be supplied separately from the lid 220 and access door 240 for ease of storage. The body 200 is slightly tapered towards the base to allow a number of body parts to be stacked together during storage. Prior to the vessel being used the body and the lid may be quickly assembled by engaging the lid and the body in a snap-fit operation which makes subsequent separation difficult.

Various modifications and variations could be made to the described vessels without departing from the ambit of the present invention. For example, access to the vessel is not limited to a pivotally sliding or hingeing door. Access may be provided by means of a linearly sliding door for example with means to temporarily and permanently lock the door. Moreover, the vessel need not be circular in section, for example, square or hexagonal sections with unreleasably inter-locking rims may be provided to form the vessel. Further, the barbed lugs and corresponding slots on the rims of the vessel parts of the first embodiment could be replaced by a bayonet-type arrangement for example which, once assembled, cannot readily be separated, or alternatively a screw-threaded arrangement could be utilized in conjunction with some means such as inter-engaging locking projections to prevent the two parts, once screwed together, from being separated, the essence of the invention being that the two parts should seal together efficiently and once assembled should not be readily separable. Furthermore, a carrying handle might by provided, par ticularly on larger sized containers, and might by hinged so as not to impede access to the opening, with the handle when in operative position arranged further to lock the two container parts together for secure transportation. Furthermore, whilst different securing and sealing means have been utilized in the three embodiments hereinbefore described, the three embodiments could be modified so as to utilize basically the same securing and sealing means.

**Claims**

1. A vessel for use in the disposal of surgical waste, the said vessel comprising first and second parts with one at least of said parts constituting a container and the other of said parts constituting a closure for said container, said first and second parts being adapted to be secured to each other so as in use to be substantially incapable of accidental detachment one from the other, and the closure part having an access opening providing direct access to the container part when the two parts are secured to each other and having furthermore a releasable closure for said access opening.

2. A vessel as claimed in claim 1 wherein the two parts of the vessel are adapted to be sealingly secured to each other so as to inhibit leakage of liquid waste from the assembled container via the juncture of the two parts, and wherein the releasable closure for the access opening is furthermore adapted to effect a sealing of the access opening so as to inhibit leakage of liquid waste from the assembled and closed container via the access opening.

3. A vessel as claimed in claim 2 wherein the two parts of the vessel have surfaces which are adapted to face seal with each other when the two parts are secured together.

4. A vessel as claimed in claim 3 wherein the face sealing surfaces of the two parts are urged together in the assembled container by means of the co-operation between a first formation provided on the first container part and a second and generally complementary formation provided on the second part.

5. A vessel as claimed in claim 4 wherein the said formations comprise a plurality of projections and a plurality of holes for receiving said projections when the two container parts are assembled together.

6. A vessel as claimed in claim 5 wherein the two vessel parts further comprise co-operating sealing flanges.

7. A vessel as claimed in claim 4 wherein a peripheral rim of one vessel part is adapted to engage in snap-fit manner with a peripheral recess of the other vessel part for securing and sealing together the two vessel parts.

8. A vessel as claimed in any of the preceding claims wherein one at least of the two parts of the vessel is so configured that a plurality of such parts can be stacked together in a nested fashion.

9. A vessel as claimed in any of the preceding claims wherein locking means are associated with said releasable closure for enabling the same to be locked in closed condition, the locking means being such as to inhibit accidental opening of the releasable closure once it has been locked closed.

10. A vessel as claimed in any of the preceding claims which is formed wholly of material that can be destroyed by incineration.

0267776

FIG.1.

10

66    68    66
72
62
60
72
60                    62
64                    72
68                    68
12
68
66    62    66

FIG.2.

70    10
62
64    76
72
78
68    66
12

FIG.3.

FIG.4.

FIG.5.

FIG.6.

0267776

0267776

FIG.7.

FIG.8.

130·01·88

0267776

FIG.10.

FIG.9.

*FIG.11.*